Europäisches Patentamt

European Patent Office

Office européen des brevets

(11) Publication number: **0 239 900 B1**

# EUROPEAN PATENT SPECIFICATION

(45) Date of publication of patent specification: **19.11.92**  (51) Int. Cl.5: **C07H  15/04**

(21) Application number: **87104242.0**

(22) Date of filing: **23.03.87**

(54) **Process for producing colour-stable industrial-grade methyl glucoside.**

(30) Priority: **02.04.86 DE 3611035**

(43) Date of publication of application:
**07.10.87 Bulletin  87/41**

(45) Publication of the grant of the patent:
**19.11.92 Bulletin  92/47**

(84) Designated Contracting States:
**AT BE CH DE ES FR GB IT LI NL SE**

(56) References cited:
**GB-A- 1 139 110**
**US-A- 3 565 885**
**US-A- 3 928 318**

(73) Proprietor: **CPC INTERNATIONAL INC.**
**International Plaza P.O. Box 8000**
**Englewood Cliffs New Jersey 07632(US)**

(72) Inventor: **Lebuhn, Rolf, Dr.**
**Randstrasse 97**
**W-4150 Krefeld(DE)**
Inventor: **Feldmann, John, Dr.**
**Avenue du General de Gaulle, 49**
**B-1050 Bruxelles(BE)**
Inventor: **Koebernick, Hubert, Dr.**
**Grüner Dyk 85**
**W-4150 Krefeld(DE)**

(74) Representative: **Lederer, Franz, Dr. et al**
**Lederer, Keller & Riederer Patentanwälte**
**Lucile-Grahn-Strasse 22**
**W-8000 München 80(DE)**

EP 0 239 900 B1

## Description

The invention relates to a process for producing a colour-stable industrial-grade methyl glucoside with a reduced content of process-derived discolouring impurities, in particular unconverted glucose, in which glucose is reacted with excess methanol at an elevated temperature with exclusion, as far as possible, of water and in the presence of an acid catalyst. The invention also relates to colour-stable industrial-grade methyl glucoside obtainable by such process, and to its use as starting or intermediate product for the manufacture of chemical products, in particular polyurethanes.

Like practically all glucosides of lower aliphatic mono-, di- and triols, methyl glucoside has long been known as has its production by reacting glucose with methanol in the presence of acid catalysts and its many uses as starting material or intermediate product for the production of a large number of industrial chemicals. Such chemicals include melamine resins, emulsifiers and detergents, in which long-chain alkyl glucosides may be incorporated as tensides and short-chain alkyl glucosides as stabilisers for the phases, for reducing the viscosity (EU-PS 136844) and as solubilisers. In addition, in certain polyether polyols derived from methyl glucoside are outstandingly suitable as intermediates for the production of polyurethanes (US patents 3277076 and 4359573 and DDR-Patent 140670).

Although alkyl glucosides in general and methyl glucoside in particular are notoriously alkali stable, their production on an industrial scale for applications which involve, as in the production of polyether polyols and processing into polyurethane foams, further reaction under alkaline conditions, presents a problem not so far satisfactorily solved. This arises because the reaction of glucose with an alcohol, and in particular methanol, in the presence of an acid catalyst according to known methods invariably yields a reaction product mixture with poor stability in the presence of alkalis, caused by a relatively high content of unconverted glucose and side reaction products. This problem occurs whether a homogeneous or a heterogeneous acid catalyst is used and/or the reaction equilibrium is shifted in favour of glucoside formation e.g. by using starch or other maltopolysaccharide instead of glucose as starting material or by adding an entrainer such as xylene, as has been proposed for synthesising $C_{3-5}$-alkyl glucosides (DE-OS 3232791, US patents 2606186, 2390509, 3346558 and 4024290, DE-OS 2035381 and Euro patent 35589).

For the above reason industrial-grade methyl glucoside cannot be used directly for the most important applications but only after a troublesome, costly purifying and stabilising treatment with bases (US 3450690) or basic anion exchange resins (US 3565885) which involve considerable losses, and/or fractional purification by crystallisation (US 2606186). Even if the discolouration during production is checked by acid condensation, e.g. by adding hydroxy polycarboxylic acids to the reaction batch (GB 2132202) , so as to reduce the formation of pigments and thereby provide certain improvement in the colour of the crude reaction product with consequent need for less purifying effort, the colour stability of the purified product in the presence of alkalis is by no means improved.

It is therefore an object of the invention to provide an improved process of the type described which overcomes the disadvantages of the prior art and which allows an industrial scale production of methyl glucoside which can be used as a starting or intermediate material for further reaction under alkaline conditions, in particular for the production of polyurethanes, without being first subjected to troublesome, costly stabilizing and purifying treatments involving loss of product.

According to the invention a process for producing colour-stable, industrial-grade methyl glucoside with a reduced content of process derived discolouring impurities, in particular unreacted glucose, which is produced by reacting excess methanol with glucose at an elevated temperature with exclusion as far as possible of water and in the presence of an acid catalyst is characterized in that glucose is reacted in two stages with methanol and at least one higher-boiling alcohol to give a glucoside mixture containing at least 60 w/w% methylglucoside and at most 0.3 w/w% glucose, measured as DE (dextrose equivalent) value, said higher-boiling alcohol having a boiling point higher than water or its azeotrope with water and if an inert entrainer is also present, of any azeotrope formed from the entrainer and water and/or higher-boiling alcohol which comprises either

a) reacting glucose in the first stage with methanol to give a reaction product mixture containing at most 20 w/w% glucose, based on dry substance, and subsequently, optionally after separation of unreacted methanol and water, reacting in the second stage the reaction product mixture with higher-boiling alcohol until the glucose content of the reaction mixture, measured as DE value, is below 0.3 w/w%, based on dry substance or,

b) reacting glucose in the first stage with higher-boiling alcohol with water continuously being separated by, in particular, azeotropic distillation, until the glucose content of the reaction mixture, measured as DE value, is below 0.2 w/w%, based on dry substance, and in the second stage reacting said reaction product, after separation of unreacted higher-boiling alcohol, with methanol until the methyl glucoside

2

content of the reaction product mixture is at least 60 w/w%, based on dry substance.

The industrial-grade methyl glucosides obtained according to the process of the invention are at least equivalent in terms of alkali stability to the best industrial-grade methyl glucosides obtainable according to known methods with the troublesome, costly and loss-involving purifying and/or upgrading treatments which are required for stabilising the crude reaction product mixture under alkaline conditions. They are also definitely superior to the only industrial-grade methyl glucoside presently available on the market, the latter being produced by reacting glucose in the presence of an acid catalyst with methanol until the dynamic reaction equilibrium has been reached, thereby yielding a crude product mixture containing typically

45 - 50% methyl-alpha-D-glucopyranoside,

approx. 25% methyl-beta-D-glucopyranoside,

20 - 25% oligosaccharides,

5 - 10% methyl-alpha, beta-glucofuranosides

and 1 - 5% glucose,

from which industrial-grade methyl-alpha-D-glucopyranoside with a DE value of about 0.5% is separated by fractional crystallisation.

While a homogenous catalyst may be used in the process of the invention, it is preferable to employ in at least one of the two stages a heterogeneous, preferably solid, acid catalyst which, upon completion of the reaction, can easily be removed and, optionally after regenerating, be re-used several times, and which has proved to be particularly suitable in the methylation stage.

Preferred solid acid catalysts are acidic, preferably inorganic molecular sieves and in particular strongly acidic cation exchange resins, of which highly cross-linked macroporous resins such as AMBERLYST 15, LEWATIT SPC 118 and slightly cross-linked gel resins such as LEWATIT SC 104 have been found to be particularly active catalysts (AMBERLYST and LEWATIT are trademarks).

It has been discovered that the catalyst concentration has a considerable influence on the ratio of alpha- and beta-glucosides which are formed. When the catalyst concentration is lowered, the proportion of beta-glucoside rises. This effect is probably due to a catalysis of the anomerising reaction in which glucoside that has already formed reacts again with alcohol molecules with inversion at the anomeric center, the beta-glucosides which are less stable reacting at a higher rate than the alpha-glucosides. In the process of the invention this effect may be used to advantage to produce industrial-grade methyl glucoside with a relatively low proportion of glucoside of the higher-boiling alcohol. This is done by selecting the conditions in the first stage, if methanol is reactant, so as to promote the formation of alpha-glucoside and, if the higher-boiling alcohol is reactant, so as to promote the formation of beta-glucoside.

A high proportion of alpha-glucoside in the product of the first stage inhibits a transglucosidising reaction in the second stage, and is therefore of advantage if the reaction with methanol takes place in the first stage and transglucosidation the second stage is an undesired side reaction. A high proportion of beta-glucoside promotes the transglucosidation reaction which, if the reaction with methanol takes place in the second stage, is the desired main reaction and so is preferred in this embodiment of the process of the invention. It should be noted that the influence of the concentration of heterogeneous catalysts on the ratio of alpha- to beta-glucosides in the reaction product mixture which has been brought to light by this invention, is new and surprising, considering that the alpha/beta ratios for varying acid concentrations are described in prior art as being constant (Euro patent 35589).

The reaction can also be influenced significantly in various respects by an appropriate selection of an excess of the alcohol used (methanol or higher boiling alcohol), a high alcohol excess having not only the expected effect of reducing the amount in the product of oligosaccharide formed by side reactions, but also of raising the yield of monomeric (ie. DP1) alkyl glucoside (in combination with the other measures of the process of the invention) to values of up to 95%, referred to glucose used, which are yields substantially above the levels given for known processes. Moreover, it surprisingly allows the ratio of alpha- to beta-glucoside to be selectively influenced, since the beta-glucoside portion in the reaction product mixture rises as the alcohol excess is increased.

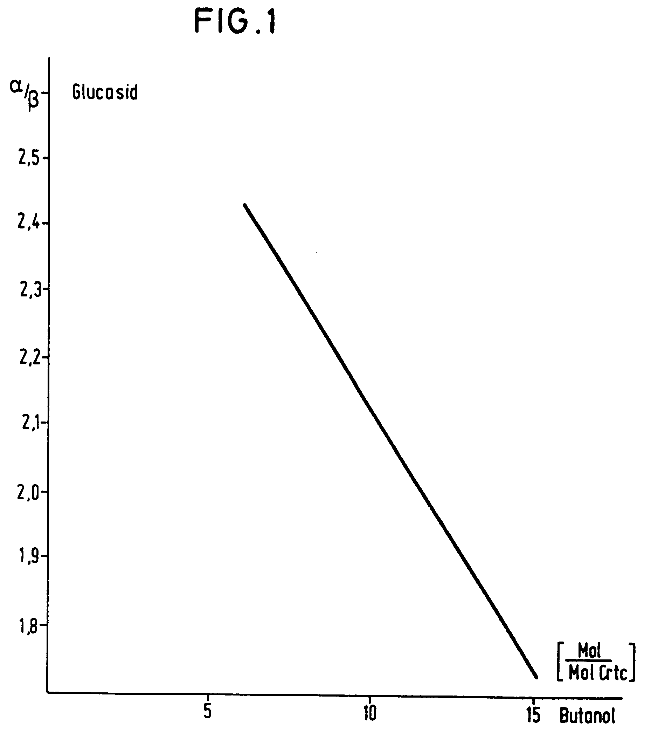

The dependence of the ratio of alpha-glucoside to beta-glucoside upon the molar ratio of alcohol to glucose is depicted in Figure 1 of the drawings for the reaction of glucose with butanol at 110°C in the presence of an acid cation exchange resin. A largely similar dependence is observed for methanol and other lower alcohols, other catalysts and/or other reaction temperatures and/or reaction times. For the alcohol excess, alcohol amounts of 3 to 20 moles per mole of glucose or transglucosidable glucoside have proved to be particularly advantageous.

During the reaction with the higher-boiling alcohol any accumulating reaction water is preferably continuously removed from the reaction mixture by distillation in order to promote glucoside formation. It is therefore recommended to use higher-boiling alcohols of the type that form with water an azeotrope which

3

is self-separating upon condensation, and/or to add to the reaction mixture an inert entrainer such as benzene or toluene, which forms an azeotrope with water and, possibly, with the higher-boiling alcohol.

Higher-boiling alohols preferred for the purposes of the invention are propanol, ethylene glycol and in particular butanol.

Although water impairs the glucosidation reaction and the highest possible degree of dryness should therefore be maintained during operation, water need not be totally excluded in the process of the invention, since, as is surprisingly shown by the example of reaction with butanol, neither the required reaction time nor the process control are unduly impaired by water when present in the batch at a concentration of up to 10 w/w%. Thus the butanol phase which forms by separation of the condensed azeotrope and which contains 20% water may be recycled to the reaction mixture without drying if a high butanol excess is used and the butanol/water azeotrope is continuously distilled off.

Water contents of more than 10% in the reaction medium however prolong the reaction time considerably.

According to a preferred embodiment of the invention a solid, preferably inorganic water-adsorbing agent which is practically insoluble in the reaction medium is added in at least one, preferably the first, stage of reaction where a comparatively large amount of reaction water accumulates. This water-adsorbing agent promotes the reaction by binding water which cannot be removed by distillation, and may after completion of the reaction easily be separated from the reaction product, and then dried and re-used.

Particularly preferred water-adsorbing agents are inorganic, acidic, molecular sieves which also act as catalysts.

Since drying is of advantage particularly in the final phase of the reaction and in cases where the water content of the reaction mixture cannot be kept low by distillation, a solid water-adsorbing agent is preferably added in the methylation stage. If methylation is the first of the two reaction stages characterising the process of the invention it has been found advantageous not to add the water-adsorbing agent until the reaction has reached an advanced stage and is slowed down by the approach to the dynamic equilibrium and the accumulation of reaction water. The water-adsorbing agent is therefore added to the reaction medium preferably during the final phase of the methylation step when a methyl glucoside content of at least 65, preferably at least 75 and more preferably at least 80 w/w%, referred to dry substance (without catalyst), has been reached.

It should be noted that the process of the invention can be carried out not only in batches, but also continuously and the catalyst and/or the water-adsorbing agent may thus be arranged in one or several bed-(s) through which the reaction mixture is passed, instead of being dispersed in the reaction medium.

According to a preferred embodiment of the invention which produces particularly alkali and colour-stable, yet low-cost industrial-grade methyl glucoside in high yields, based on glucose consumed, the glucose is in the first stage reacted with methanol until the reaction product mixture contains at least 80, preferably at least 90, and more preferably at least 95 w/w% methyl glucosides referred to dry substance, such reaction being preferably performed under conditions which promote the formation of alpha-methyl glucoside rather than of beta-methyl glucoside. The reaction product mixture thus obtained is then in the second stage reacted with the higher-boiling alcohol until the (residual) glucose content of the reaction product mixture is at most 0.3, preferably at most 0.2, and more preferably not more than 0.15 w/w%, referred to dry substance and measured as DE value.

Another possibility of advantageously modifying the process of the invention consists of reacting the glucose with methanol in the first stage and recovering from the reaction product mixture of the first stage, by concentrating and precipitating, an intermediate product featuring a higher total content of methyl glucosides and higher ratio of alpha- to beta-methyl glucoside for use in the second reaction stage. By this technique the transglucosidation of methyl glucoside with higher-boiling alcohol which takes place as a side reaction in the second stage reaction with the higher-boiling alcohol can be largely limited.

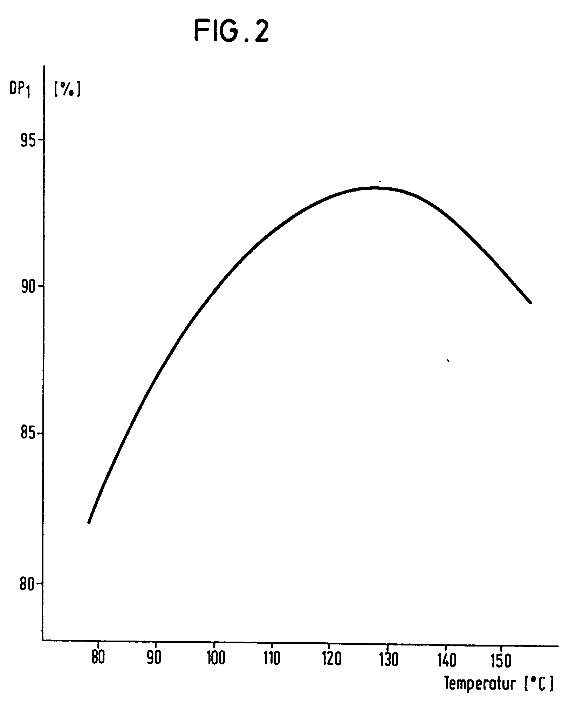

Another parameter by which the reaction can be controlled is the reaction temperature which allows selective adjustment of both the proportion of monomeric glucopyranosides and the ratio of the alpha- to beta-glucoside. The rising reaction temperature causes a higher rate of glucose conversion while the transformation of the furanosides developed under kinetically controlled conditions into pyranosides at first rises steeply and then drops again due to an increased formation of oligosaccharides or, more precisely, oligomer glucosides. The reaction of glucose with butanol shows the pattern depicted in Figure 2 of the drawing, which is largely similar to that shown by other alcohols. The ratio of alpha- to beta-glucoside increases as the temperature rises. It should also be noted that for the reaction with higher-boiling alcohols the reaction temperature may be adjusted within a relatively wide range by an appropriate selection of the pressure in the reactor, whereas for the reaction with methanol it is in most cases recommended to apply normal pressure or elevated pressure under reflux.

4

The examples illustrate the two variants of the process of the invention, i.e. further glucosidation of methyl glucoside starting material and transglucosidation with methanol of a high-quality glucoside of a higher-boiling alcohol , using butanol as the example of a higher-boiling alcohol which is preferred for the process of the invention.

In the examples the colour stability of the product under alkaline conditions and under heat is determined as follows:

Colour stability under alkaline conditions:

The alkali stability is measured in aqueous 70% solution. In the presence of 7% potassium hydroxide the solution is heated for one hour to 80ºC, whereupon the colour of the sample is determined according to the Gardner scale.

Colour stability under heat:

The heat stability is measured in aqueous 70% solution. The sample is exposed uncovered for four hours to a temperature of 160ºC. After restoring to the original weight with distilled water, the colour of the simple is determined according to the Gardner scale.

Example 1

In a 1-liter three-necked flask with reflux condenser and stirrer 240 g anhydrous glucose, 540 g methanol and 60 g of a highly acidic cation exchanger (AMBERLYST 15) were mixed at approx. 1000 RPM and heated under reflux. After 10 hours 40 g of a molecular sieve of 3 A pore size were added and the mixture was heated under stirring for another two hours under reflux. The resulting nearly colourless solution was filtered while still hot, and the filtrate was concentrated to dryness. The resulting methyl glucoside (250 g) showed a content of reducing sugars, measured as DE (dextrose equivalent) of 5.1%.

Before the second reaction stage the crude glucoside was transferred to a flask with drip funnel, stirrer, condenser and thermometer. After adding 1,050 g butanol and 30 g AMBERLYST 15 the solution was heated under stirring to a temperature in the liquid of 90ºC and the butanol/water azeotrope distilled off at a vacuum of 300 mbars, while the liquid volume in the reaction flask was kept constant by adding butanol dropwise. After a reaction time of 1.5 hours the solution was filtered while hot, neutralised with 1N caustic soda solution and the butanol distilled off; the small amounts of methanol present in the distillate may be separated from the butanol by distillation.

The syrupy residue was dissolved in water and decolourised with the aid of activated carbon. After evaporation of the water a DE value of 0.07 was measured for the final methyl glucoside product.

| Colour stability under alkaline conditions: | 7.0 |
| Colour stability under heat: | 0.4. |

According to HPLC analysis 65% methyl glucoside and 35% butyl glucoside were present in the final product. The proportion of monomeric glucopyranoside ($DP_1$) was 87%.

Example 2

In a reactor with reflux condenser and stirrer 5 kg anhydrous glucose, 4.5 kg methanol and 1.5 g of highly acid cation exchange resin (LEWATIT SP 11 dry) were mixed and heated under reflux. After a reaction time of 12 hours the product showed a DE value of 8.0 and was removed from the catalyst by filtration. The solution was concentrated by distilling off 2.5 kg methanol and the residue was cooled down to room temperature. A white precipitate (1.3 kg) resulted, which was separated by centrifuging and subjected to an analysis which showed a DE value of 1.5 and a content of methyl-alpha-D-glucopyranoside of 92%. 250 g of this product were transferred to a flask with drip funnel, stirrer, condenser and thermometer and blended with 1.050 g butanol and 25 g cation exchange resin (LEWATIT SPC 118). The solution was heated under stirring and at a temperature in the liquid of 80 - 90ºC and under a vacuum of 300 mbars a butanol/water azeotrope was distilled off, while butanol was continuously added dropwise to the reaction batch in order to keep the liquid volume constant. After a reaction time of two hours the solution was filtered while hot, neutralised with 1N caustic soda solution and butanol was distilled off.

The residue was dissolved in water and decolourised with the aid of activated carbon. After concentrating a DE value of 0.08 was found in the final product.

| Colour stability under alkaline conditions: | 7.2 |
| Colour stability under heat: | 0.4. |

According to HPLC analyses 94% methyl glucoside and 6% butyl glucoside were present in the final product. The proportion of monomeric glucopyranoside ($DP_1$) was 94%.

Example 3

In a three-necked flask with reflux condenser and stirrer 240g anhydrous glucose, 540 g methanol and 70 g acid cation exchange resin (AMBERLYST 15) were mixed and heated under reflux. After a reaction time of 10 hours the solution was filtered while hot and concentrated to dryness. The DE value of the resulting crude methyl glucoside (250 g) was 9.5.

In a flask with drip funnel, stirrer, condenser and thermometer the glucoside was blended with 880 g ethylene glycol and 37 g cation exchange resin (AMBERLYST 15). At a temperature in the liquid of 75°C and under a vacuum of 3 mbars the reaction water was separated from the stirred batch by distillation; any co-distilled ethylene glycol being replaced dropwise. After a reaction time of 1.5 hours the solution was filtered while hot, neutralised with 1N caustic soda solution and the alcohol was distilled off. The residue was dissolved in water and decolourised with the aid of activated carbon. The final product showed a DE value of 0.10.

| Colour stability under alkaline conditions: | 8.5 |
| Colour stability under heat: | 0.8. |

According to HPLC analyses 71% methyl glucoside and 29% hydroxyethyl glucoside were present in the final product. The proportion of monomeric glucopyranoside ($DP_1$) was 95%.

Example 4

In a flask with drip funnel, stirrer, condenser and thermometer methyl glucoside (250 g) produced as in Example 3 was mixed with 850 n-propanol and 45 g cation exchange resin (AMBERLYST 15). At a temperature in the liquid of 83°C and under a vacuum of 700 mbars propanol/water azeotrope was distilled off, the volume of the reaction batch being kept constant by dropwise addition of n-propanol. After a reaction time of 1.5 hours the solution was separated from the cation exchanger by filtering, neutralised with 1N caustic soda solution and the alcohol was distilled off. The residue was dissolved in water, decolourised with the aid of activated carbon and concentrated to dryness. The DE value of the resulting methyl glucoside was found to be 0.05.

| Colour stability under alkaline conditions: | 6.3 |
| Colour stability under heat: | 0.3. |

According to HPLC analyses 78% methyl glucoside and 22% propyl glucoside were present in the final product. The proportion of monomer ie glucopyranoside ($DP_1$) was 95%.

Example 5

In a flask with drip funnel, stirrer, condenser and thermometer 80g anhydrous glucose, 300 g n-butanol and 10 g of a highly acid cation exchange resin (AMBERLYST 15) were mixed at approx. 1000 RPM and heated to a temperature in the liquid of 90°C. Under a vacuum of 300 mbars the reaction water was distilled off as azeotrope and the liquid volume in the reaction flask kept constant by dropwise addition of n-butanol. After a reaction time of six hours the solution was filtered while hot and the butanol distilled off. The butyl glucoside (100 g) obtained as residue showed a DE value of 0.10 and contained 53% n-butyl-alpha-D-glucopyranoside and 37% n-butyl-beta-D-glucopyranoside.

In the second stage the butyl glucoside was blended with 170 g dried methanol and 30 g cation exchange resin (AMBERLYST 15) and with stirring heated under reflux. After a reaction time of 10 hours the solution was filtered while hot, neutralised with 1N caustic soda solution, the methanol distilled off at normal pressure and the n-butanol at reduced pressure. The residue was dissolved in water and decolourised with the aid of activated carbon. The methyl glucoside thus obtained as final product showed a DE value of 0.15.

| | |
|---|---|
| Colour stability under alkaline conditions: | 10.0 |
| Colour stability under heat: | 0.9. |

According to HPLC analyses 67% methyl glucoside and 33% butyl glucoside were present in the final product. The proportion of monomeric glucopyranoside ($DP_1$) was 84%.

**Claims**

1. A process for producing colour-stable, industrial-grade methyl glucoside with a reduced content of process-derived discolouring impurities, in particular unreacted glucose which comprises reacting excess methanol with glucose at an elevated temperature with exclusion as far as possible of water and in the presence of an acid catalyst characterised in that glucose is reacted in two stages with methanol and at least one higher-boiling alcohol to give a glucoside mixture containing at least 60 w/w% methyl glucoside and at most 0.3 w/w% glucose, measured as DE (dextrose equivalent) value, said higher-boiling alcohol having a boiling point higher than water or its azeotrope with water and, if an inert entrainer is also present, of any azeotrope formed from the entrainer and water and/or higher-boiling alcohol which comprises either

   a) reacting in the first stage glucose with methanol to give a reaction product mixture containing at most 20 w/w% glucose, based on dry substance, and subsequently, optionally after separation of unreacted methanol and water, reacting in the second stage the reaction product mixture with higher-boiling alcohol until the glucose content of the reaction mixture, measured as DE value, is below 0.3 w/w% based on dry substance, or,

   b) reacting glucose in the first stage with higher-boiling alcohol with water continuously being separated by, in particular, azeotropic distillation, until the glucose content of the reaction mixture, measured as DE value, is below 0.2 w/w% based on dry substance, and in the second stage reacting said reaction product, after separation of unreacted higher-boiling alcohol, with methanol until the methyl glucoside content of the reaction product mixture is at least 60 w/w% based on dry substance.

2. The process of claim 1, characterised in that in at least one stage, in particular the methylation stage, a heterogeneous, acid catalyst, preferably one that is solid under reaction condition, is present.

3. The process of claim 2, characterised in that the catalyst is a strongly acidic, cation exchange resin, in particular a highly cross-linked macroporous resin or a slightly cross-linked gel resin, or an acidic, preferably inorganic, molecular sieve.

4. The process of any one of claims 1 to 3, characterised in that alcohol (methanol and/or higher-boiling alcohol) is used at a molar ratio to glucose or transglucosidable glucoside of from 3:1 to 20:1.

5. The process of any one of claims 1 to 4, characterised in that the higher-boiling alcohol used is propanol, ethylene glycol or, preferably, butanol.

6. The process of any one of claims 1 to 5, characterised in that in at least one, preferably the first stage of the process there is present a solid, preferably inorganic, water adsorbing agent which is practically insoluble in the reaction mixture.

7. The process of claim 6, characterised in that the water absorbing agent is an inorganic, acidic molecular sieve.

8. The process of claim 6 or claim 7, characterised in that the water adsorbing agent is added to the reaction mixture in the final phase of the methylation step when a methyl glucoside content of at least

EP 0 239 900 B1

65, preferably at least 75 and more preferably at least 80 w/w%, referred to dry substance, has been reached.

9. The process of any one of claims 1 to 8, characterised in that the glucose is in the first stage reacted with methanol until the reaction product mixture contains at least 80, preferably at least 90, and more preferably at least 95 w/w% methyl glucoside, referred to dry substance, and that the reaction product mixture is then reacted with the higher-boiling alcohol until the glucose content of the reaction product mixture referred to dry substance and measured as DE value, is at most 0.3, preferably at most 0.2, and more preferably not more than 0.15 w/w%.

10. The process of any one of claims 1 to 9 characterised in that the glucose is in the first stage reacted with methanol and by concentrating and precipitating an intermediate product with an increased content of methyl glucoside is obtained from the reaction product mixture of the first stage prior to the second stage.

**Patentansprüche**

1. Verfahren zur Herstellung eines farbfesten Methylglukosides von technischer Qualität mit einem herabgesetzten Gehalt an verfahrensbedingten entfärbenden Verunreinigungen, insbesondere nicht umgesetzter Glukose, welches Verfahren die Umsetzung eines Ueberschusses an Methanol mit Glukose bei einer erhöhten Temperatur unter Ausschluss, so weit wie möglich, von Wasser und in Gegenwart eines sauren Katalysators umfasst, dadurch gekennzeichnet, dass Glukose in zwei Stufen mit Methanol und mindestens einem höhersiedenden Alkohol umgesetzt wird, um ein Glukosidgemisch zu ergeben, das mindestens 60 % Gew./Gew. Glukose, gemessen als DE (Dextroseäquivalent)-Wert, ergibt, wobei der höhersiedende Alkohol einen höheren Siedepunkt als Wasser oder des Azeotrops mit Wasser und, falls auch ein inerter Mitläufer vorhanden ist, als jedes aus dem Mitläufer und Wasser und/oder höhersiedendem Alkohol gebildete Azeotrop aufweist, wobei das Verfahren entweder:
   a) die Umsetzung in der ersten Stufe von Glukose mit Methanol, um ein Reaktionsproduktegemisch zu ergeben, das höchstens 20 Gewichtsprozent Glukose, basierend auf der Trockensubstanz, enthält, und anschliessende Umsetzung, gegebenenfalls nach Abtrennung von nichtumgesetztem Methanol und Wasser, des Reaktionsproduktegemisches in der zweiten Stufe mit höhersiedendem Alkohol bis der Gehalt des Reaktionsgemisches, gemessen als DE-Wert, unter 0,3 %, Gewicht/Gewicht, basierend auf der Trockensubstanz, liegt, oder
   b) die Umsetzung von Glukose in der ersten Stufe mit höhersiedendem Alkohol, wobei Wasser kontinuierlich, insbesondere durch azeotrope Destillation, abgetrennt wird, bis der Glukosegehalt des Reaktionsgemisches, gemessen als DE-Wert, unterhalb 0,2 % Gew./Gew., basierend auf der Trockensubstanz, liegt, und in der zweiten Stufe die Reaktion dieses Reaktionsproduktes, nach Abtrennung von nicht-umgesetztem höhersiedendem Alkohol, mit Methanol, bis der Methylglukosidgehalt des Reaktionsproduktegemisches mindestens 60 % Gew./Gew., basierend auf der Trockensubstanz beträgt, umfasst.

2. Verfahren nach Anspruch 1, dadurch gekennzeichnet, dass in mindestens einer Stufe, insbesondere in der Methylierungsstufe, ein heterogener saurer Katalysator, vorzugsweise ein solcher, der unter der Reaktionsbedingung fest ist, zugegen ist.

3. Verfahren nach Anspruch 2, dadurch gekennzeichnet, dass der Katalysator ein stark saures Kationenaustauscherharz, insbesondere ein hochvernetztes, makroporöses Harz oder ein schwachvernetztes Gelharz oder ein saures, vorzugsweise anorganisches Molekularsieb ist.

4. Verfahren nach einem der Ansprüche 1 bis 3, dadurch gekennzeichnet, dass Alkohol (Methanol und/oder höhersiedender Alkohol) in einem molaren Verhältnis zu Glukose oder transglukosidalem Glukosid von 3:1 bis 20:1 verwendet wird.

5. Verfahren nach einem der Ansprüche 1 bis 4, dadurch gekennzeichnet, dass der verwendete höhersiedende Alkohol Propanol, Aethylenglykol oder vorzugsweise Butanol ist.

6. Verfahren nach einem der Ansprüch 1 bis 5, dadurch gekennzeichnet, dass in mindestens einer, vorzugsweise der ersten Stufe des Verfahrens ein festes, vorzugsweise anorganisches, wasseradsorbie-

8

rendes Mittel, welches im Reaktionsgemisch praktisch unlöslich ist, zugegen ist.

7. Verfahren nach Anspruch 6, dadurch gekennzeichnet, dass das wasseradsorbierende Mittel ein anorganisches, saures Molekularsieb ist.

8. Verfahren nach Anspruch 6 oder 7, dadurch gekennzeichnet, dass das wasseradsorbierende Mittel dem Reaktionsgemisch in der Endphase der Methylierungsstufe zugesetzt wird, wenn ein Methylglukosidgehalt von mindestens 65, vorzugsweise von mindestens 75 und noch bevorzugterweise von mindestens 80 % Gew./Gew., bezogen auf die Trockensubstanz, erreicht ist.

9. Verfahren nach einem der Ansprüche 1 bis 8, dadurch gekennzeichnet, dass die Glukose in der ersten Stufe mit Methanol umgesetzt wird, bis das Reaktionsproduktegemisch mindestens 80, vorzugsweise mindestens 90 und noch bevorzugterweise mindestens 95 % Gew./Gew. Methylglukosid, bezogen auf die Trockensubstanz, enthält, und dass das Reaktionsproduktegemisch dann mit dem höhersiedenden Alkohol umgesetzt wird, bis der Glukosegehalt des Reaktionsproduktegemisches, bezogen auf die Trockensubstanz und gemessen als DE-Wert, höchstens 0,3 , vorzugsweise höchstens 0,2 und noch bevorzugterweise nicht mehr als 0,15 % Gew./Gew. beträgt.

10. Verfahren nach einem der Ansprüche 1 bis 9, dadurch gekennzeichnet, dass die Glukose in der ersten Stufe mit Methanol umgesetzt wird und dass durch Konzentrieren und Ausfällen ein Zwischenprodukt mit erhöhtem Gehalt an Methylglukosid aus dem Reaktionsproduktegemisch der ersten Stufe vor der zweiten Stufe erhalten wird.

**Revendications**

1. Procédé de production de méthyl-glucoside de qualité technique, de couleur stable, avec une teneur réduite en impuretés modifiant la couleur provenant du procédé, en particulier de glucose n'ayant pas réagi, qui est produit par réaction de méthanol en excès avec du glucose à une température élevée en l'absence d'eau aussi complète que possible et en présence d'un catalyseur acide, caractérisé en ce que du glucose est amené à réagir en deux étapes avec du méthanol et au moins un alcool de plus haut point d'ébullition pour former un mélange de glucosides contenant au moins 60 % en poids/poids de méthyl-glucoside et au maximum 0,3 % en poids de glucose, mesuré d'après la valeur ED (équivalent en dextrose), cet alcool de point d'ébullition élevé ayant un point d'ébullition supérieur à celui de l'eau ou de son azéotrope avec l'eau et, si une substance inerte d'entraînement est également présente, supérieur à celui de tout azéotrope formé entre la substance d'entraînement et l'eau et/ou l'alcool de point d'ébullition élevé, qui comprend
   a) la réaction de glucose dans la première étape avec du méthanol pour former un mélange de produits réactionnels contenant au maximum 20 % en poids/poids de glucose, sur base sèche, puis, facultativement après séparation du méthanol n'ayant pas réagi et de l'eau, la réaction dans la seconde étape du mélange de produits réactionnels avec l'alcool de point d'ébullition élevé jusqu'à ce que la teneur en glucose du mélange réactionnel, mesurée d'après la valeur ED, soit inférieure à 0,3 % en poids/poids sur base sèche, ou bien
   b) la réaction de glucose dans la première étape avec un alcool de point d'ébullition élevé, avec séparation ininterrompue de l'eau, en particulier par distillation azétropique, jusqu'à ce que la teneur en glucose du mélange réactionnel, mesurée d'après la valeur ED, soit inférieure à 0,2 % en poids/poids sur base sèche, et dans la seconde étape, la réaction dudit produit réactionnel, après séparation de l'alcool de point d'ébullition élevé n'ayant pas réagi, avec du méthanol jusqu'à ce que la teneur en méthyl-glucoside du mélange formant le produit réactionnel soit au moins égale à 60 % en poids/poids, sur base sèche.

2. Procédé suivant la revendication 1, caractérisé en ce qu'un catalyseur acide hétérogène, de préférence un catalyseur qui est solide dans les conditions réactionnelles, est présent dans au moins une étape.

3. Procédé suivant la revendication 2, caractérisé en ce que le catalyseur est une résine d'échange cationique fortement acide, en particulier une résine macroporeuse fortement réticulée ou une résine gélifiée légèrement réticulée ou un tamis moléculaire acide, de préférence inorganique.

4. Procédé suivant l'une quelconque des revendications 1 à 3, caractérisé en ce qu'un alcool (méthanol et/ou alcool de plus haut point d'ébullition) est utilisé dans un rapport molaire avec le glucose ou le glucoside susceptible de transglucosidation de 3:1 à 20:1.

**5.** Procédé suivant l'une quelconque des revendications 1 à 4, caractérisé en ce que l'alcool de point d'ébullition élevé utilisé est le propanol, l'éthylène-glycol ou de préférence le butanol.

**6.** Procédé suivant l'une quelconque des revendications 1 à 5, caractérisé en ce que dans au moins une étape du procédé, de préférence la première, un agent solide de préférence inorganique adsorbant l'eau, qui est pratiquement insoluble dans le mélange réactionnel, est présent.

**7.** Procédé suivant la revendication 6, caractérisé en ce que l'agent adsorbant l'eau est un tamis moléculaire acide inorganique.

**8.** Procédé suivant la revendication 6 ou la revendication 7, caractérisé en ce que l'agent adsorbant l'eau est ajouté au mélange réactionnel dans la phase finale de l'étape de méthylation lorsqu'une teneur en méthyl-glucoside d'au moins 65 % en poids/poids, de préférence d'au moins 75 % en poids/poids et notamment d'au moins 80 % en poids/poids, sur base sèche, a été atteinte.

**9.** Procédé suivant l'une quelconque des revendications 1 à 8, caractérisé en ce que le glucose est amené à réagir dans la première étape avec du méthanol jusqu'à ce que le mélange constituant le produit réactionnel contienne au moins 80, de préférence au moins 90 et notamment au moins 95 % en poids/poids de méthyl-glucoside, sur base sèche, et en ce que le mélange constituant le produit de réaction est ensuite amené à réagir avec l'alcool de point d'ébullition élevé jusqu'à ce que la teneur en glucose du mélange constituant le produit de réaction, sur base sèche et mesurée d'après la valeur ED, soit au plus égale à 0,3 % en poids/poids, de préférence au plus égale à 0,2 % en poids/poids et ne dépasse pas notamment 0,15 % en poids/poids.

**10.** Procédé suivant l'une quelconque des revendications 1 à 9, caractérisé en ce que le glucose est amené à réagir dans la première étape avec du méthanol et un produit intermédiaire à teneur élevée en méthyl-glucoside est obtenu par concentration et précipitation à partir du mélange constituant le produit réactionnel de la première étape, avant la seconde étape.

# FIG.1

# FIG.2